# EUROPÄISCHE PATENTSCHRIFT

(11) **EP 2 557 940 B1**
(45) Veröffentlichungstag und Bekanntmachung des Hinweises auf die Patenterteilung: **14.10.2015**
(21) Anmeldenummer: 11714212.5
(22) Anmeldetag: 28.03.2011
(51) Int. Cl.: A23K 1/12, A23K 1/14, A23K 1/16, A61K 31/575, A61K 36/54, A61P 1/00, A61P 33/00, A23K 1/18, A61K 45/06, A61K 36/15, A61K 36/575

(54) **TIERFUTTERZUSATZ MIT ANTIMIKROBIELLER UND LEISTUNGSFÖRDERNDER WIRKUNG**
ANIMAL FEED ADDITIVE HAVING AN ANTIMICROBIAL AND GROWTH-PROMOTING EFFECT
ADDITIF D'ALIMENTATION ANIMALE AYANT UNE ACTION ANTI-MICROBIENNE ET TONIFIANTE

(30) Priorität: 15.04.2010 AT 6112010
(43) Veröffentlichungstag der Anmeldung: 20.02.2013
(73) Patentinhaber: Neufeld, Klaus, 2532 Heiligenkreuz (AT)
(72) Erfinder: Neufeld, Klaus, 2532 Heiligenkreuz (AT)
(74) Vertreter: Beer & Partner Patentanwälte KG
(86) Internationale Anmeldenummer: PCT/AT2011/000152
(87) Internationale Veröffentlichungsnummer: WO 2011/127499

(56) Entgegenhaltungen:
- WO-A1-2008/058698
- CN-A- 1 253 816
- CN-A- 101 390 572
- CN-A- 101 543 261
- CN-A- 101 595 947
- JP-A- 1 075 428

## Beschreibung

Die Erfindung betrifft einen Tierfutterzusatz mit antimikrobieller und leistungsfördernder Wirkung, welcher Lignocellulose-hältiges, Eisenionen-bindendes Material aus Holz, Holzfasern bzw. Rinden und Rindenfasern von Pinusarten in Kombination mit einem pflanzlichen, antimikrobiellen Wirkstoff, welcher als Extrakt bzw. Material aus Pflanzen der Gattung Magnolia vorliegt, enthält. Die Erfindung betrifft weiters die Verwendung des erfindungsgemäßen Tierfutterzusatzes zur Herstellung eines Tierfuttermittels.

Seit gut 20 Jahren werden in der landwirtschaftlichen, tierischen Produktion Wirkstoffe pflanzlichen Ursprungs als Alternative zu den lange in Gebrauch gewesenen antimikrobiellen, chemischen Leistungsförderern eingesetzt. Unter "leistungsfördernde Wirkung" versteht der Fachmann im Nutztierbereich folgende Effekte, die bei physiologisch gesunden Tieren zu verzeichnen sind: Verbesserung der Tageszunahmen bei Masttieren; Verbesserung der Futterverwertung (Verhältnis der Futteraufnahme zur Leistung wie z.B. Gewichtszunahme, Milchleistung, Eizahl); Verbesserung der Zuchtleistung wie z.B. größere Wurfzahl, höhere Einzelgewichte und bessere Überlebensrate der Jungtiere oder Verringerung des Remontierungszeitraumes; Erhöhung der Milchleistung bei milchproduzierenden Tieren. Aufgrund der Tatsache, dass pflanzliche Substanzen, meistens Extrakte, in ihrer Gewinnung oftmals teurer als die früher verwendeten antimikrobiellen, chemischen Leistungsförderer sind, ergibt sich aus ökonomischer Sicht die Notwendigkeit, die Wirkung dieser Substanzen zu optimieren, um deren Einsatz für den Landwirt zufriedenstellend zu gestalten.

Aus der WO 2008/058698 A1 sind leistungsfördernde Zusatzstoffe für die tierische Ernährung, welche eine Lignocellulose-hältige Faserformulierung sowie einen Isochinolinalkaloid-hältigen Ballaststoff enthalten, bekannt.

Aus der EP 1 075 428 A ist weiters ein antimikrobieller Zusatzstoff für Fischfutter, welcher Rindenmaterial der Pflanzengattungen Magnolia und linnamomum enthält, bekannt.

Aus der CN 101 543 261 A ist ein Tierfutterzusatz mit antibakterieller und antiviraler Wirkung bekannt, welcher das Wachstum von Hühnern fördert. Dieser Tierfutterzusatz enthält Rindenmaterial aus Magnolia officinalis mit einer Reihe anderer medizinisch wirksamer Pflanzenrinden und Wurzeln.

Aus der CN 101 595 947 ist ein Tierfutterzusatz für Hasen bekannt, welcher das Auftreten einer durch Staphylococcus aureus ausgelösten Brustentzündung verhindert. Der Tierfutterzusatz enthält Magnolia officinalis und Süßholz.

In der CN 101 390 572 ist ein antivirales Tierfuttermittel beschrieben, welches unter anderem geringfügige Mengen an Magnolia und Kiefernnadeln enthält. Es handelt sich hierbei um eine auf der traditionellen chinesischen Medizin basierenden Mischung, welche eine Vielzahl anderer Plfanzen, Kräuter, Schalen, Samen und dergleichen enthält.

Die bekannten Tierfutterzusätzstoffe mit teilweise antiviraler bzw. antibakterieller Wirkung enthalten zwar neben Lignicellulose-hältigen Materialien auch Extrake bzw. Material aus der Gattung der Magnolia officinalis, allerdings oftmals in geringfügigen Mengen oder aber in Kombination mit einer Vielzahl anderer faseriger bzw. pflanzlicher Stoffe. Somit ist die Nacharbeitbarkeit aus verfahrenstechnischer Sicht oftmals schwierig bzw. lässt sich der jeweilige Wirkmechanismus bei erkrankten Tieren nicht zur Gänze nachvollziehen.

Hier will die Erfindung Abhilfe schaffen.

Erfindungsgemäß wird ein Tierfutterzusatz der eingangs genannten Art vorgeschlagen, welcher dadurch gekennzeichnet ist, dass der Anteil an Lignocellulose-hältigem, Eisenionen-bindenden Material, zumindest 10 Gew.-% bezogen auf 100 Gew.-% Tierfutterzusatz ist.

Die meisten Bakterien benötigen für ihr Wachstum als Substrat unter anderem Eisen. Es konnte nachgewiesen werden, dass bestimmte Lignocellulose-hältige Substanzen, wie bestimmte Holzarten, insbesondere aber Rinde, bevorzugterweise von Koniferen, speziell Pinusarten, Eisen binden können.

Der erfindungsgemäße Tierfutterzusatz bewirkt somit den Synergieeffekt, dass sowohl durch den antimikrobiellen Wirkstoff als auch durch die Bindung von für das Bakterienwachstum notwendigen Eisen das Wachstum von Bakterien zusätzlich gehemmt wird. Daher ist es möglich, pflanzliche, antimikrobielle Wirkstoffe beim erfindungsgemäßen Tierfutterzusatz in geringeren Dosierungen als bisher einzusetzen und somit eine Kostenreduktion zu erreichen.

Vorteilhafte Ausgestaltungen des erfindungsgemäßen Tierfutterzusatzes sind gemäß Ansprüche 2 bis 4 offenbart.

Die Erfindung betrifft weiters die Verwendung des erfindungsgemäßen Tierfutterzusatzes zur Behandlung oder vorbeugenden Behandlung von Tieren gegen den Befall durch krankheitserregenden Mikroben.

Die Erfindung wird nunmehr anhand von vorteilhaften Ausführungen zur Durchführung der Erfindung näher erläutert.

Als besonders geeignete Kombination haben sich Produkte aus Lignocellulose-haltiger Holzfaser bzw. Rindenfaser insbesondere von Pinusarten in Kombination mit den Substanzen Magnolol und Honokiol erwiesen. Diese Substanzen kommen insbesondere in Magnoliaarten bspw. der Magnolia acuminata, Magnolia biondii, Magnolia denudata, Magnolia grandiflora, Magnolia kobus, Magnolia obovata, Magnolia officinalis, Magnolia sprengeri, Magnolia tripetala, Magnolia virginiana, Magnolia glauca vor. Ein derartiges Produkt kann bspw. aus Holzmehl (Holzfaser) bzw. Rindenmehl (Rindenfaser) in Kombination mit Pflanzenmaterial der genannten Magnoliaarten bzw. Extrakten daraus bestehen, wobei bspw. der Gehalt an Magnolol und Honokiol in einer derartigen Mischung bspw. 0,0001% - 90% betragen kann, der Anteil des Lignocellulosematerials 99,9999% - 10% betragen kann, besonders bevorzugt 0,1% - 20% betragen kann, der Anteil des Lignocellulosematerials 80% - 99,9% betragen kann.

Besonders bevorzugt wird Magnoliarinde verwendet, die in reiner, ausschließlich vermahlener Form oder als Extrakt eingesetzt werden kann.

Der Extrakt kann prinzipiell durch unterschiedliche Extraktionsmittel wie z.B. durch Wasserdampfdestillation, Extraktion mit Wasser oder mit Alkohol-Wasser Gemischen, alkoholische Extraktion, lipophile Extraktion oder superkritische CO₂ Extraktion hergestellt werden. Diese Extrakte können in flüssiger oder getrockneter (pulverförmig) Form vorliegen.

Die Wirkstoffe aus Magnolia officinalis werden vorzugsweise aus der Rinde mittels superkritischer CO₂ Extraktion gewonnen. Das Rohmaterial wird gewaschen und bei 50°C bis zu einem Trockensubstanzgehalt von mindestens 91% getrocknet und anschließend zerkleinert. Im Extraktor wird das so aufbereitete Rohmaterial mit superkritischem CO₂ als Extraktionsmittel bei einer Durchflussrate von 1.200 bis 1.400 Liter pro Stunde für einen Zeitraum von 3,5 Stunden bei einem Druck von 25 bis 30 MPa und einer Temperatur von 35 bis 40°C extrahiert. Der Extrakt wird anschließend in Ethanol aufgenommen und auf den gewünschten Wirkstoffgehalt eingestellt.

Wie aus den nachfolgenden Untersuchungen zu entnehmen ist, eignet sich als Eisenionen-bindendes Lignocellulosematerial, insbesondere Kiefernrinde, Kiefernholz und Fichtenholz. Mit Lärchenholz, das ebenfalls getestet wurde, konnten die gewünschten synergistischen Effekte nicht erzielt werden. Überraschenderweise konnte dieser synergistische Effekt nur mit den genannten Rinden und Holzarten erreicht werden.

| | | | |
|---|---|---|---|
| Testmaterial | Kiefernrinde | Magnoliaextrakt | Kiefernrinde + Magnoliaextrakt 1:1 |
| Breite des Hemmhofes | keine Hemmwirkung | 5 mm | 10 mm |
| Testmaterial | Kiefernholzmehl | Magnoliaextrakt | Kiefernholzmehl + Magnoliaextrakt 1:1 |
| Breite des Hemmhofes | keine Hemmwirkung | 5 mm | 8 mm |
| Testmaterial | Fichtenholzmehl | Magnoliaextrakt | Fichtenholzmehl + Magnoliaextrakt 1:1 |
| Breite des Hemmhofes | keine Hemmwirkung | 5 mm | 6 mm |
| Testmaterial | Lärchenholz | Magnoliaextrakt | Lärchenholz + Magnoliaextrakt 1:1 |
| Breite des Hemmhofes | keine Hemmwirkung | 5 mm | 5 mm |
| Keim | Staphylococcus aureus | | |

Ebenso sind Kombinationen einzelner antimikrobieller Wirkstoffe miteinander in Kombination mit der beschriebenen Lignocellulosequelle möglich. So hat sich eine Kombination von Pflanzenmaterial der Magnoliaarten und Cinnamomumarten, als sinnvoll erwiesen.

Das erfindungsgemäße Produkt kann entweder alleine oder in Kombination mit anderen Füll- und Hilfsstoffen der Futtermittelverarbeitung in Form eines Futtermittelzusatzes, einer Vormischung oder eines Ergänzungsfuttermittels dem Tierfutter beigemischt werden, oder vom Landwirt direkt dem Futter zugegeben werden. Weiters kann das Produkt als orale Formulierung dem Tier auch direkt eingegeben werden, oder bspw. über das Trinkwasser appliziert werden. Die Einmischrate eines derartigen Produktes beträgt üblicherweise zwischen 0,1 g/t Futtermittel und 100kg/t, bevorzugterweise zwischen 1 g/t und 50kg/t Futtermittel.

Folgende Rationsbeispiele werden angeführt:
Beispielrezeptur Schweinemastfutter; Angaben in %

| Vit. Mineral. Prämix | Soja HP | Gerste | Weizen | Mais | Weizenkleie | erfindungsgemäßer Futtermittelzusatz |
|---|---|---|---|---|---|---|
| 3 | 19 | 30,9 | 20 | 20 | 7 | 0,1 =1.000ppm |

Beispielrezeptur Schweinemast Flüssigfütterung Universalmastfutter; Angaben in %

| Vit. Mineral. Prämix | Soja 44 | Maiskornsilage (35% Feuchte) | Wasser | Faserkonzentrat | erfindungsgemäßer Futtermittelzusatz |
|---|---|---|---|---|---|
| 0,9 | 6,2 | 27,05 | 65,4 | 0,4 | 0,05 = 500ppm |

Beispielrezeptur Ferkelfutter; Angaben in %

| Vit. Mineral. Prämix | Soja 44 | Gerste | Weizen | Mais | Säuremix | erfindungsgemäßer Futtermittelzusatz |
|---|---|---|---|---|---|---|
| 4 | 21,5 | 29,8 | 22 | 22 | 0,5 | 0,2 = 2.000ppm |

Beispielrezeptur Broilerfutter; Angaben in %

| | |
|---|---|
| Maisschrot | 30,4 |
| Weizenschrot | 20 |
| Fett | 4,4 |
| Sojavollbohnenschrot | 12,0 |
| Sojaextraktionsschrot (44% CP) | 4,9 |
| Sojaextraktionsschrot (48% CP) | 19,0 |
| Fischmehl (64% CP) | 2,0 |
| Rapsextraktionsschrot | 3,0 |
| DL-Methionin | 0,15 |
| Dicalciumphosphat | 1,85 |
| Calciumcarbonat | 0,94 |
| Natriumchlorid | 0,26 |
| Vit. Mineralstoff Prämix | 1,0 |
| erfindungsgemäßer Futtermittelzusatz | 0,1 = 1.000ppm |

Der eingangs erwähnte Synergieeffekt wird nunmehr anhand von Fütterungsversuchen bei einer Ferkelaufzucht bzw. bei der Aufzucht einer Broilermast erläutert, siehe Tabellen 1 und 2.

**Tabelle 1: Fütterungsversuch Ferkelaufzucht**

| | Kontrolle | Versuch^{*)} | Versuch % |
|---|---|---|---|
| Tiere (Stück) | 46 | 42 | |
| Versuchsdauer (Tage) | 34 | 34 | |
| Anfangsgewicht pro Tier (kg) | 12,35 | 12,57 | 101,8 |
| Endgewicht pro Tier (kg) | 31,54 | 33,19 | 105,2 |
| Tageszunahme (g) | 565 | 606 | 107,3 |
| Futterverbrauch pro Tier (kg) | 32,83 | 33,17 | 101,0 |
| Futterverwertung | 1,71 | 1,61 | 94,2 |
| Kosten/kg Aufmast (€) | 0,544 | 0,511 | 93,9 |

| | | | |
|---|---|---|---|
| *) 2.000 ppm erfindungsgemäßer Futterzusatz im Futter der Versuchsgruppe | | | |

**Tabelle 2: Fütterungsversuch Broilermast**

| | Kontrolle | Versuch^{#)} | Versuch % |
|---|---|---|---|
| Tiere (Stück) | 42.065 | 40.242 | |
| Masttage | 30 | 30 | |
| Futterverbrauch pro Tier (kg) | 2,539 | 2,549 | 100,4 |
| Wasserverbrauch pro Tier (I) | 4,50 | 4,40 | 97,8 |
| Wasser: Futter | 1,77 | 1,73 | 97,7 |
| Tageszunahme (g) | 51,2 | 52,5 | 102,5 |
| Futterverwertung | 1,656 | 1,621 | 97,9 |
| Gewinn/Tier (€) | 0,146 | 0,163 | 111,6 |
| Mastkennzahl (MKZ) | 304 | 318 | 104,6 |

| | | | |
|---|---|---|---|
| #) 2.000 ppm erfindungsgemäßer Futterzusatz im Futter der Versuchsgruppe | | | |

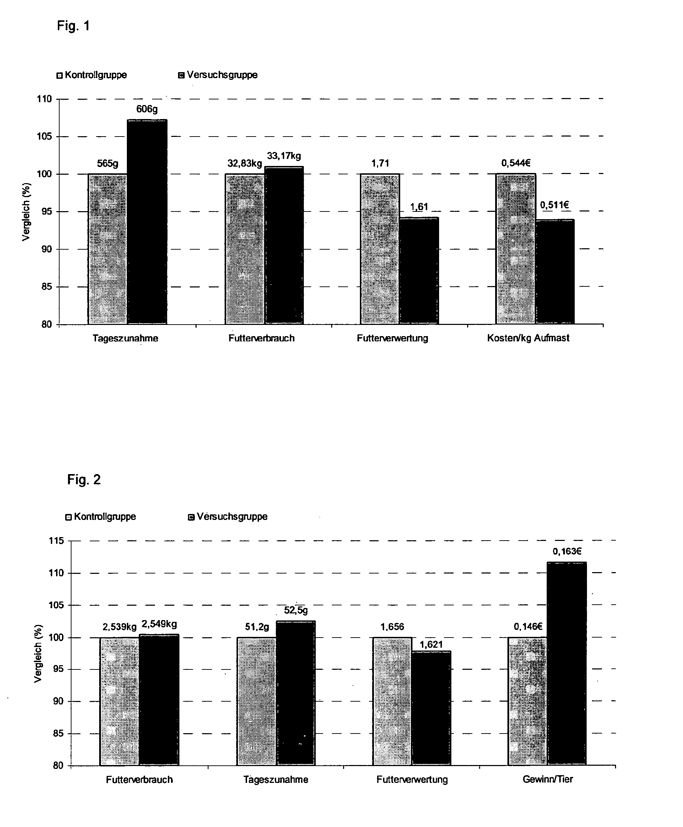

In Tabelle 1 werden die Bedingungen für einen Fütterungsversuch von Ferkeln aufgelistet, wobei die Kontrollgruppe 46 Tiere und die Versuchsgruppe 42 Tiere umfasst. Die Versuchsdauer betrug in beiden Fällen 34 Tage, wobei, wie aus dem in Fig. 1 dargestellten Balkendiagramm zu der Versuchsführung gemäß Tabelle 1 zu ersehen ist, dass bei den Tieren der Versuchsgruppe jeweils eine zusätzliche Tageszunahme von mehr als 40 g Körpergewicht verzeichnet wurde. Überraschender Weise war der Futterverbrauch sowohl bei der Kontrollgruppe als auch bei jener Versuchsgruppe von Tieren, an welche der erfindungsgemäße Futterzusatz verfüttert worden ist, identisch. Allerdings konnte die Futterverwertung durch die Tiere der jeweiligen Versuchsgruppe drastisch reduziert werden, was auf den Bestandteil der Lignocellulose-hältigen Substanzen in Kombination mit dem antimikrobiellen Wirkstoff zurückzuführen ist.

Ähnliche Ergebnisse konnten bei Fütterungsversuchen von Broilern, siehe dazu Tabelle 2, verzeichnet werden. Die in Tabelle 2 angeführten Versuchsbedingungen wurden anhand eines Balkendiagramms gemäß Fig. 2 erzielt dargestellt.

Ähnliche positive Ergebnisse wie bei der Wachstumsleistung von landwirtschaftlichen Nutztieren konnten mit der erfindungsgemäßen Substanz auch bei eierlegenden Nutztieren in Hinblick auf Anzahl der gelegten Eier, Qualitätssortierung der Eier, Futterverwertung und Ausfälle der Tiere beobachtet werden. Ein ebensolcher Effekt wurde auch bei Fischen im Hinblick auf Wachstumsintensität und Ausfallmenge verzeichnet.

Besonders überraschende Effekte konnten beim Wiederkäuer, wie beispielsweise der Milchkuh, dem Mastrind, Ziegen und Schafen, durch Verfüttern des erfindungsgemäßen Tierfutterzusatzes erzielt werden. Bei diesen Tierarten, die über einen längeren Zeitraum hohe Leistungen erbringen müssen, ist es nämlich besonders schwierig eine Leistungssteigerung zu erreichen. In verschiedenen Versuchen bei Milchkühen konnte ein positiver Einfluss auf die Milchmengenleistung, in einzelnen Fällen aber auch auf die Milchqualitätsparameter, wie Proteingehalt und Milchfettgehalt, nachgewiesen werden.

In einem Versuch bei Milchkühen der Rasse Schwarzbunte wurden insgesamt 60 Tiere in drei Gruppen zu je 20 Tieren eingeteilt, und die Milchleistung über eine Laktationsperiode beobachtet. Die Milchleistung wird als Gruppendurchschnitt in Kilogramm pro Tier und Jahr angegeben. Die Tiere der Nullkontrollgruppe erhielten eine übliche Milchviehration ohne leistungsfördernde Zusätze, die Tiere der Positivkontrollgruppe erhielten einen marktüblichen pflanzlichen Leistungsförderer, dessen Wirkung auf ätherischen Ölen basiert, und in einer Konzentration von 1g pro Tier und Tag in Verbindung mit dem üblichen Kraftfutter gegeben wurde. In der Versuchsgruppe erhielten die Tiere 30 g des erfindungsgemäßen Tierfutterzusatzes pro Tier und Tag in Verbindung mit üblichem Kraftfutter.

| | Nullkontrolle | Positivkontrolle | Versuchsgruppe |
|---|---|---|---|
| ätherische Öle | - | 1g/Tier/Tag | - |
| erfindungsgem. Sub. | - | - | 30g/Tier/Tag |
| Milchleistung | 8.520kg/Tier/Jahr | 8.670kg/Tier/Jahr | 8.830kg/Tier/Jahr |

Bei Mastrindern wurde eine Verbesserung der Tageszunahme nachgewiesen. In unterschiedlichen Versuchen, sowohl in vitro als auch an der pansenfistulierten Kuh, konnte mit dem erfindungsgemäßen Tierfutterzusatz nachgewiesen werden, dass im Pansen die Nährstoffverdauung, insbesondere die Kohlenhydratverdauung, verzögert werden kann. Dies ist ein besonders wünschenswerter Effekt, da einerseits die überschießende Utilisation von Nährstoffen durch die Pansenflora zur Bildung größerer Mengen minderwertigen Bakterienproteins führt, andererseits die rasche Verwertung von Kohlenhydraten im Pansen zu einem schnellen und unerwünschten Abfall des pH-Wertes im Pansen führt.

Im Rusitec (künstlicher Pansen) wurde nachgewiesen, dass ein Zusatz der erfindungsgemäßen Substanz die pH-Wert Senkung nach der Zugabe von Kohlenhydraten (Kraftfuttergabe) verringert wird. Im Rusitec Versuch wurde hierzu einmal dem Rusitec System eine Kohlenhydratquelle zugegeben und der pH-Wert gemessen; in einem zweiten Versuchsdurchgang wurde gemeinsam mit der Kohlenhydratquelle die erfindungsgemäße Substanz zugesetzt. In der Kontrollgruppe erreichte der pH-Wert bis 5,4, in der Versuchsgruppe fiel er lediglich bis 6,1 ab.

Der erfindungsgemäße Tierfutterzusatz ist daher auch zur Prophylaxe der Pansenazidose bei Wiederkäuern wie der Milchkuh, dem Mastrind, Ziegen und Schafen, geeignet.

In einem Versuch mit pansenfistulierten Kühen wurden jeweils drei Versuchstiere mit kohlenhydratreicher Ration ohne Zusatz gefüttert; drei weitere Versuchstiere wurden mit der selben Ration, allerdings in Verbindung mit dem erfindungsgemäßen Tierfutterzusatz, in einer Menge von 30g pro Tier und Tag gefüttert. Der Versuch lief über einen Zeitraum von einer Woche. Jeweils drei Stunden nach der Kraftfuttergabe wurde Pansensaft entnommen und der pH-Wert bestimmt. Die in der Tabelle angegebenen Werte sind die Durchschnittswerte der jeweiligen Gruppe jeweils nach der ersten und der zweiten täglichen Kraftfuttergabe.

| | pH-Wert 1 | pH-Wert 2 |
|---|---|---|
| Kontrollgruppe | 5,4 | 5,7 |
| Versuchsgruppe | 6,1 | 6,3 |

Des Weiteren kann der erfindungsgemäße Tierfutterzusatz zur vorbeugenden Behandlung von Tieren gegen den Befall durch krankheitserregende Mikroben verwendet werden.

Nekrotisierende Enteritis, ausgelöst durch Clostridium perfringens, stellt beim Geflügel, insbesondere bei Legehennen, ein großes wirtschaftliches Problem dar. Die Symptome sind eine chronische Enteritis, verringerte Futteraufnahme, gesteigerte Wasseraufnahme, zu feuchter Kot, Veränderungen des Federkleides, Federpicken und Federfressen, Gewichtsabnahme, verringerte Legeleistung mit einer gesteigerten Varianz des Eigewichts, verkürzte Nutzungsdauer, Infektanfälligkeit sowie erhöhte Mortalität aufgrund von Erschöpfung oder Sekundärinfektionen wie etwa E. coli Infektionen.

Die Krankheit erweist sich als langwierig und therapieresistent. Die Verabreichung von Antibiotika ist problematisch, da die Krankheitssymptome nach Absetzen der Therapie wiederkommen und eine Antibiotikatherapie aufgrund der geltenden Absetzfristen einen hohen ökonomischen Schaden verursacht. Eier können während der Therapie und während der Absetzfrist nicht verwertet werden, aufgrund der kurzen Mastdauer von Broilern (Masthühnern) können diese nicht therapiert werden. Andere mögliche Therapieansätze, wie Veränderung der Futterzusammensetzung, Anpassung des Rohfaser- oder Rohproteingehaltes im Futter, Verabreichung essentieller Aminosäuren oder Fettsäuren, Anpassung des Vitamin- oder Mineralstoffgehaltes im Futter, Zusatz von organischen Säuren etc., haben sich als ineffizient erwiesen.

In einem Legehennenbetrieb, in dem der behandelnde Tierarzt Symptome einer Clostridien-Enteritis diagnostiziert hatte, wurde einer von drei betroffenen Herden die erfindungsgemäße Substanz in einer Dosierung von 1.000 g pro Tonne Futtermittel verabreicht. In der Folge wurde eine nachhaltige Verbesserung der Tiergesundheit bis zum vollständigen Fehlen jeglicher Symptome festgestellt. Die Herde erreichte die volle Legeleistung, während die anderen beiden betroffenen, aber mit herkömmlichen diätetischen Methoden behandelten, Herden (es wurden dem Futter essentielle Aminosäuren und Fettsäuren sowie organische Säuren zugesetzt) eine weitere Verschlechterung des Krankheitsbildes aufwiesen. Unter Verwendung der erfindungsgemäßen Substanz lag die Mortalitätsrate in diesem Legehennenbetrieb bei 3,8 Prozent, während in den anderen Herden, die mit herkömmlichen diätetischen Maßnahmen therapiert wurden, die Tierausfälle bei 8,1 Prozent lagen.

Somit kann mit Hilfe des erfindungsgemäßen Tierfutterzusatzes eine Reihe überraschender Effekte erzielt werden, welche auf die Synergien zwischen dem verwendeten, aus Pflanzen gewonnen antimikrobiellen Wirkstoff und dem Eisenionen-bindenden, Lignocellulose-hältigen Material zurückzuführen ist.

| **Ferkelaufzucht** | **Kontrolle** | **V M** | **V LC8** | **V LC11** | **V LC50** |
|---|---|---|---|---|---|
| Tiere (Stück) | 46 | 42 | 44 | 42 | 42 |
| Versuchsdauer (Tage) | 34 | | | | |
| Anfangsgewicht pro Tier (kg) | 12,35 | 12,51 | 12,34 | 12,60 | 12,57 |
| Endgewicht pro Tier (kg) | 31,54 | 31,96 | 31,72 | 33,14 | 33,19 |
| Tageszunahme (g) | 565 | 572 | 570 | 604 | 606 |
| Futterverbrauch pro Tier (kg) | 32,83 | 32,86 | 32,79 | 33,19 | 33,17 |
| Futterverwertun g | 1,71 | 1,69 | 1,69 | 1,62 | 1,61 |
| Ausfälle (Stück Tiere) | 7 | 6 | 7 | 2 | 1 |

Es handelt sich hierbei um den Fütterungsversuch Ferkelaufzucht von Seite 6, Tabelle 1, ergänzt durch weitere Versuchsgruppen zur Verdeutlichung. Der Versuch wurde als Feldversuch unter erhöhtem Infektionsdruck mit praxisüblichen Stallkeimen durchgeführt. Allen Versuchsgruppen wurde die in der jeweiligen Gruppe zur Verwendung gelangte Testsubstanz (siehe unten) in einer Dosierung von 2.000ppm supplementiert.

| **Broilermast** | **Kontrolle** | **V M** | **V LC8** | **V LC11** | **V LC50** |
|---|---|---|---|---|---|
| Tiere (Stück) | 42.065 | 42.097 | 40.184 | 42.120 | 40.242 |
| Masttage | 30 | | | | |
| Futterverbrauch pro Tier (kg) | 2,539 | 2,539 | 2,540 | 2,544 | 2,549 |
| Wasserverbrau ch pro Tier (I) | 4,50 | 4,50 | 4,60 | 4,40 | 4,40 |
| Wasser: Futter | 1,77 | 1,77 | 1,81 | 1,73 | 1,73 |
| Tageszunahme (g) | 51,2 | 51,4 | 51,1 | 52,4 | 52,5 |
| Tierausfälle (%) | 3,79 | 3,57 | 3,59 | 0,78 | 0,88 |

Es handelt sich hierbei um den Fütterungsversuch Broilermast von Seite 6, Tabelle 2, ergänzt durch weitere Versuchsgruppen zur Verdeutlichung. Der Versuch wurde als Feldversuch unter erhöhtem Infektionsdruck mit praxisüblichen Stallkeimen durchgeführt. Allen Versuchsgruppen wurde die in der jeweiligen Gruppe zur Verwendung gelangte Testsubstanz (siehe unten) in einer Dosierung von 2.000ppm supplementiert.
V M: Testsubstanz bestehend aus 100% Magnoliaextrakt
V LC8: Testsubstanz bestehend aus 8% Lignocellulose (Kiefernrinde) und 92% Magnoliaextrakt
V LC11: Testsubstanz ist erfindungsgemäßer Futterzusatz bestehend aus 11% Lignocellulose (Kiefernrinde) und 89% Magnoliaextrakt
V LC50: Testsubstanz ist erfindungsgemäßer Futterzusatz bestehend aus 50% Lignocellulose (Kiefernrinde) und 50% Magnoliaextrakt

## Patentansprüche

1. Tierfutterzusatz mit antimikrobieller und leistungsfördernder Wirkung, welcher ein lignocellulose-haltiges, Eisenionen-bindendes Material aus Holz, Holzfasern bzw. Rinde und Rindenfasern von Pinusarten in Kombination mit einem pflanzlichen, antimikrobiellen Wirkstoff, welcher als Extrakt bzw. Material aus Pflanzen der Gattung Magnolia vorliegt, enthält, **dadurch gekennzeichnet, dass** der Anteil an Lignocellulose-hältigem, Eisenionen-bindenden Material zumindest 10 Gew.-% bezogen auf 100 Gew.-% Tierfutterzusatz ist.

2. Tierfutterzusatz nach Anspruch 1, **dadurch gekennzeichnet, dass** der pflanzliche antimikrobielle Wirkstoff in einem Anteil von zumindest 0,0001 Gew.-% bezogen auf 100 Gew.-% Tierfutterzusatz vorliegt.

3. Tierfutterzusatz nach Anspruch 1 oder 2, **dadurch gekennzeichnet, dass** der pflanzliche, antibakterielle Wirkstoff Magnolol und/oder Honokiol ist.

4. Tierfutterzusatz nach einem der Ansprüche 1 bis 3, **dadurch gekennzeichnet, dass** die tägliche Dosierung für Nutztiere, inklusive Wiederkäuer, zwischen 1g und 100g pro Tier und Tag liegt.

5. Verwendung eines Tierfutterzusatzes nach einem der Ansprüche 1 bis 4 zur Herstellung eines Tierfuttermittels für die Behandlung oder vorbeugende Behandlung von Tieren gegen den Befall von krankheitserregenden Mikroben, **dadurch gekennzeichnet, dass** der Tierfutterzusatz in einem Bereich von 0,1 g bis 100 kg in jeweils eine Tonne Futtermittel eingemischt wird.

6. Verwendung eines Tierfutterzusatzes nach Anspruche 5, **dadurch gekennzeichnet, dass** der Tierfutterzusatz in einem Bereich von 1 g bis 50 kg in jeweils eine Tonne Futtermittel eingemischt wird.

## Claims

1. Animal feed supplement with antimicrobial and performance-enhancing action, which contains a lignocellulose-containing, iron ion-binding material from wood, wood fibres or bark and bark fibres from pine species in combination with a plant-based antimicrobial agent which is present as an extract or material from plants of the genus magnolia, **characterized in that** the proportion of lignocellulose-containing iron ion-binding material is at least 10% by weight in relation to 100% by weight animal feed supplement.

2. Animal feed supplement as claimed in claim 1, **characterized in that** the plant-based antimicrobial agent is present in a proportion of at least 0.0001% by weight, in relation to 100% by weight animal feed supplement.

3. Animal feed supplement as claimed in claim 1 or claim 2, **characterized in that** the plant-based antimicrobial agent is magnolol and/or honokiol.

4. Animal feed supplement as claimed in one of claims 1 to 3, **characterized in that** the daily dose for livestock, including ruminants, is between 1 g and 100 g per animal per day.

5. Use of an animal feed supplement as claimed in one of claims 1 to 4 for the manufacture of an animal feed supplement for the treatment or prophylactic treatment of animals against infestation by pathogenic microbes, **characterized in that** the animal feed supplement is mixed into the animal feed in an amount of 0.1 g to 100 kg at any one ton of animal feed.

6. Use of an animal feed supplement as claimed in claim 5, **characterized in that** the animal feed supplement is mixed into the animal feed in an amount of 1 g to 50 kg at any one ton of animal feed.

## Revendications

1. Additif alimentaire pour animaux à effet antimicrobien et stimulant les performances, lequel contient une matière contenant de la lignocellulose, liant les ions ferriques en bois, en fibres de bois ou en écorce et fibres d'écorce de types de pins, en combinaison avec une principe actif végétal antimicrobien qui se présente sous forme d'extrait ou de matière de végétaux de l'espèce Magnolia, **caractérisé en ce que** la part en matière contenant de la lignocellulose, liant les ions ferriques est d'au moins 10 % en poids en rapport à 100 % en poids d'additif alimentaire pour animaux.

2. Additif alimentaire pour animaux selon la revendication 1, **caractérisé en ce que** le principe actif végétal antimicrobien se présente dans une part d'au moins 0,0001 % en poids en rapport à 100 % en poids d'additif alimentaire.

3. Additif alimentaire pour animaux selon la revendication 1 ou la revendication 2, **caractérisé en ce que** le principe actif végétal antibactérien est du magnolol et/ou de l'honokiol.

4. Additif alimentaire selon l'une quelconque des revendications 1 à 3, **caractérisé en ce que** le dosage journalier pour des animaux de rente, y-compris des ruminants se situe entre 1 g et 100 g par animal et par jour.

5. Utilisation d'un additif alimentaire pour animaux selon l'une quelconque des revendications 1 à 4 pour la fabrication d'un produit alimentaire pour animaux pour le traitement ou le traitement préventif d'animaux contre l'infestation de microbes pathogènes, **caractérisée en ce qu'**on ajoute par mélange l'additif alimentaire pour animaux dans un ordre de 0,1 g à 100 kg dans chaque fois une tonne de produit alimentaire pour animaux.

6. Utilisation d'un additif alimentaire pour animaux selon la revendication 5, **caractérisée en ce qu'**on ajoute par mélange l'additif alimentaire pour animaux dans un ordre de 1 g à 50 kg dans chaque fois une tonne de produit alimentaire pour animaux.
